# EUROPÄISCHE PATENTSCHRIFT

(11) **EP 1 618 908 B1**
(45) Veröffentlichungstag und Bekanntmachung des Hinweises auf die Patenterteilung: **29.11.2006**
(21) Anmeldenummer: 05009941.5
(22) Anmeldetag: 06.05.2005
(51) Int. Cl.: A61M 5/158, A61M 39/10, A61M 25/02

(54) **Kupplungsvorrichtung zum Anschliessen einer medizinischen Versorgungsleitung an einen Katheter**
Coupling device for connecting a medical feed line to a catheter
Dispositif d'accouplement pour raccorder un circuit médical d'alimentation à un cathéter

(30) Priorität: 23.07.2004 DE 202004011671 U
(43) Veröffentlichungstag der Anmeldung: 25.01.2006
(73) Patentinhaber: Clinico GmbH, 36251 Bad Hersfeld (DE)
(72) Erfinder: Heinzerling, Jörg, 36251 Bad Hersfeld (DE); Csincsura, Roland, 99817 Eisenach (DE)
(74) Vertreter: von Eichel-Streiber, Caspar

(56) Entgegenhaltungen:
- DE-A1- 10 117 285
- US-A- 6 123 690
- US-A1- 2001 053 889
- US-A1- 2002 161 332
- US-B1- 6 368 141

## Beschreibung

Die Erfindung betrifft eine Kupplungsvorrichtung zum Anschließen einer medizinischen Versorgungsleitung an einen Katheter.

In der Medizin werden in verschiedenen Bereichen zur Verabreichung von in Flüssigkeit gelösten oder suspendierten Medikamenten Katheter verwendet, durch die über eine Punktionsstelle die Medikation direkt in das Gewebe oder eine Blutbahn des Patienten verabreicht wird. Dabei muss zum Anlegen des Katheters zunächst ein Zugang zum Gewebe bzw. der Blutbahn des Patienten durch Punktion geschaffen werden. Anschließend wird an den Katheter eine Versorgungsleitung angelegt. Hierzu dienen entsprechende Kupplungsvorrichtungen die ein schnelles und sicheres Anschließen und Trennen der Versorgungsleitung an den bzw. von dem Katheter erlauben.

Bekannte derartige Kupplungsvorrichtungen, siehe z.B. US 2001/053889 A1, US 2002/161332 A1, weisen einerseits auf dem Katheter ein Katheteranschlussstück und andererseits an der Versorgungsleitung ein Kupplungsstück auf. Zum Ankuppeln bzw. Anschließen der Leitung an den Katheter wird das Kupplungsstück auf das Katheteranschlussstück aufgesetzt, wobei das Kupplungsstück mit geeigneten Mitteln dort fixiert wird, in der Regel verrastet. In dem Kupplungsstück ist dabei regelmäßig eine Kupplungskanüle in Form einer Hohlnadel angeordnet. Diese dient dazu, beim Kupplungsvorgang ein in dem Katheteranschlussstück befindliches Dichtelement (Septum) zu durchstoßen, um so den Flüssigkeitskanal zwischen der Versorgungsleitung und dem Katheter herzustellen. Diese Kupplungskanüle ist hierzu mit einer scharfen (angeschrägten) Spitze versehen, damit sie das Dichtelement durchstechen kann.

Insbesondere für Patienten, die eigenverantwortlich und eigenständig mit Kathetern und medizinischen Versorgungsleitung umgehen, wie bspw. Insulin-Patienten, ist es wichtig, derartige Kupplungsvorrichtungen so auszugestalten, dass sie einfach und fehlerfrei handhabbar sind.

Hierzu muss insbesondere eine sichere Führung der Kupplungselemente (Katheteranschlussstück und Kupplungsstück) beim Zusammenfügen der Kupplungsvorrichtung gewährleistet sein, wobei insbesondere Querkräfte vermieden werden sollen, die u.a. zu einem Verrutschen bzw. Ablösen des Katheters führen können.

Zudem ist es wünschenswert, dass der Patient bzw. sonstige Anwender einer Kupplungsvorrichtung bspw. entweder durch ein hörbares Geräusch oder durch taktiles Empfinden ein sicheres Einrasten der Kupplungsvorrichtung erkennt.

Es ist Aufgabe der vorliegenden Erfindung, eine diesen Ansprüchen genügende Kupplungsvorrichtung zu schaffen.

Diese Aufgabe wird gelöst durch eine Kupplungsvorrichtung zum Anschließen einer medizinischen Versorgungsleitung an einen Katheter mit einem an dem Katheter angeordneten Katheteranschlussstück (Hub) und einem an einem Anschlussende der medizinischen Versorgungsleitung zum Zusammenwirken mit dem Katheteranschlussstück angeordneten Kupplungsstück, wobei jeweils eines der Elemente Katheteranschlussstück und Kupplungsstück jeweils eines der paarweise zusammenwirkenden Kupplungselemente aufweist:
1. einen axial in einer Aufschubrichtung des Kupplungsstückes ausgerichteten Zapfen bzw. eine zur Aufnahme des Zapfens ausgestaltete Buchse und,
2. einen in einer parallel zur axialen Erstreckungsrichtung des Zapfens keilförmig ausgebildeten Verriegelungskeil bzw. einen Keilsitz zur formschlüssigen Aufnahme des Verriegelungskeils,
wobei der Keilsitz durch gelenkig gelagerte Formschlusselemente begrenzt ist, welche zum Lösen einer formschlüssigen Verbindung zwischen dem Verriegelungskeil und dem Keilsitz auseinander bewegbar sind und wobei an dem Element Katheteranschlussstück, bzw. Kupplungsstück, an dem der Verriegelungskeil angeordnet ist voneinander beabstandete Hebelarme angeordnet sind, die bei zusammengefügter Kupplungsvorrichtung so mit den Formschlusselementen zusammenwirken, dass sie diese bei Betätigung der Hebelarme spreizen und den Verriegelungskeil freigeben.

Die erfindungsgemäße Kupplungsvorrichtung wird durch die axiale in einer Aufschubrichtung des Kupplungsstückes ausgerichteten Zapfen und Buchse bei einem Zusammenfügen sicher geführt. Die Kombination aus Zapfen und Buchse sorgt ferner bei zusammengefügter Kupplungsvorrichtung für einen quer zu der axialen Erstreckungsrichtung dieser beiden Elemente eingehaltenen Formschluss, der eine Trennung der Kupplungsvorrichtung in einer Richtung quer zu dieser axialen Richtung verhindert. Eine Verrieglung der Kupplungsvorrichtung in der axialen Richtung wird durch den Verrieglungskeil und den entsprechenden Keilsitz gewährleistet. Ferner wird dadurch, dass der Keilsitz durch gelenkig gelagerte Formschlusselemente begrenzt ist, bewirkt, dass diese beim "Einrasten" des Keils ein hörbares Schnappgeräusch verursachen, so dass der Anwender der Kupplungsvorrichtung ein korrektes Einrasten derselben hören kann. Neben dem hörbaren Geräusch wird auch ein taktil spürbares Vibrieren ausgelöst, welches der Anwender ebenfalls wahrnehmen kann. Die gemäß Anspruch 1 weiterhin vorhandenen Hebelarme ermöglichen ein einfaches Auflösen der Verbindung, indem bei Betätigen der Hebelarme die Formschlusselemente gespreizt und damit der Keil aus dem Keilsitz freigegeben wird. Nun kann die Kupplungsvorrichtung durch axiales Abziehen des Kupplungsstückes in Richtung der Achse des Zapfens bzw. der Buchse gelöst werden.

Vorteilhafte Weiterbildungen der erfindungsgemäßen Kupplungsvorrichtung sind in den Ansprüchen 2 bis 10 angegeben.

In Anspruch 2 ist eine mögliche Konkretisierung des Zusammenspiels zwischen den Hebelarmen und den Formschlusselementen angegeben. Eine weitere konkretere Möglichkeit dieser Gestaltung ist in Anspruch 3 gegeben, wobei die Steuerzapfen eine einfache und zugleich zuverlässige Variante darstellen, die Kraftübertragung von den Hebelarmen auf die Formschlusselemente zu bewirken.

Eine Ausgestaltung der Kupplungsvorrichtung, wie sie in Anspruch 4 gekennzeichnet ist, bedingt zum einen eine zusätzliche Führung des Kupplungsstücks gegenüber dem Katheteranschlussstück, zum anderen wird durch die wie beschrieben ausgebildete Kontur der Führungsstege beim Betätigen der Hebelarme bereits die axiale Trennung unterstützt bzw. eingeleitet. Dies führt insgesamt zu einer einfachen Handhabbarkeit der Kupplungsvorrichtung auch beim Trennen derselben und vermindert insbesondere die Gefahr, dass beim Trennen unerwünschte Kräfte auf den Katheter an sich wirken, die Schmerzen beim Patienten hervorrufen oder sogar zu einer Ablösung des Katheters und damit einer Unterbrechung der Medikamentenversorgung führen könnten. Eine bevorzugte Ausgestaltung der Kupplungsvorrichtung ist in Anspruch 5 angegeben. In dieser Ausgestaltung lässt sich der Katheter besonders flach gestalten, so dass er bei der Anwendung nicht übermäßig von der Körperoberfläche des Patienten absteht und Angriffsfläche zum "Hängenbleiben" bildet.

Eine Ausgestaltung der Kupplungsvorrichtung, wie sie in Anspruch 6 vorgesehen ist, unterstützt zum einen die kompakte Bauweise des Katheters auch bei angeschlossener medizinischer Versorgungsleitung, zum anderen wird durch das Zusammenspiel zwischen der Grundplatte und den Hebelarmen eine zusätzliche Führung des Kupplungsstückes beim Verbinden mit dem Katheteranschlussstück erzielt.

Eine Ausgestaltung der Kupplungsvorrichtung gemäß Anspruch 7 bedingt zusätzlich eine kompakte und kleine Bauweise, der Kupplungsvorrichtung insgesamt. Dadurch, dass die Formschlusselemente und damit der Keilsitz in die Grundplatte integriert sind, ist es nicht erforderlich, weitere "Anbauten" an dem Katheteranschlussstück zur Ausbildung eines Keilsitzes vorzusehen.

Mit einer gemäß Anspruch 8 ausgebildeten Klebeplatte kann das Katheteranschlussstück bzw. der Katheter auf der Hautoberfläche des Patienten fixiert werden. Durch den Einschnitt in der Klebeplatte kann diese den Bewegungen der Grundplatte beim Aufspreizen der in der Grundplatte integrierten Formschlusselemente folgen.

Eine Ausgestaltung, wie sie in Anspruch 9 vorgeschlagen ist, bietet den Vorteil, dass die Kupplungsvorrichtung beim Einstechen der Kupplungskanüle in das Dichtelement anders als beim Stand der Technik mit spitzen Kupplungskanülen keine Seitenkräfte erfährt. Durch die abgerundete Penetrationsspitze der Kupplungskanüle fährt diese axial und geradeaus in das Dichtelement ein, ohne aufgrund eines seitlich angebrachten Anschliffes zur Seite gedrückt zu werden. Damit die abgerundete Kupplungskanüle das Dichtelement sicher durchdringen kann, ist Letztere vorperforiert.

Hinsichtlich der geometrischen Ausgestaltung der Buchse und des Zapfens wird die in Anspruch 10 angegebene Zylinderform mit kreisförmigem Querschnitt bevorzugt. Es sind jedoch auch Zapfen- und Buchsengeometrien mit anderen Querschnitten, dreieckig, viereckig oder polygon denkbar und möglich.

Weitere Merkmale und Vorteile der Erfindung ergeben sich aus der nachfolgenden Beschreibung eines Ausführungsbeispiels anhand der beigefügten Figuren. In den Figuren zeigen:
- Fig. 1: eine erfindungsgemäße Kupplungsvorrichtung mit Katheteranschlussstück und Kupplungsstück in getrenntem Zustand in einer perspektivischen Ansicht von oben,
- Fig. 2: eine perspektivische Ansicht von unten auf eine erfindungsgemäße Kupplungsvorrichtung mit in das Katheteranschlussstück eingerastetem Kupplungsstück (ohne Klebeplatte) und
- Fig. 3: eine erfindungsgemäße Kupplungsvorrichtung mit auf das Katheteranschlussstück aufgesetztem Kupplungsstück in einer teilweise geschnittenen Seitenschnitt.

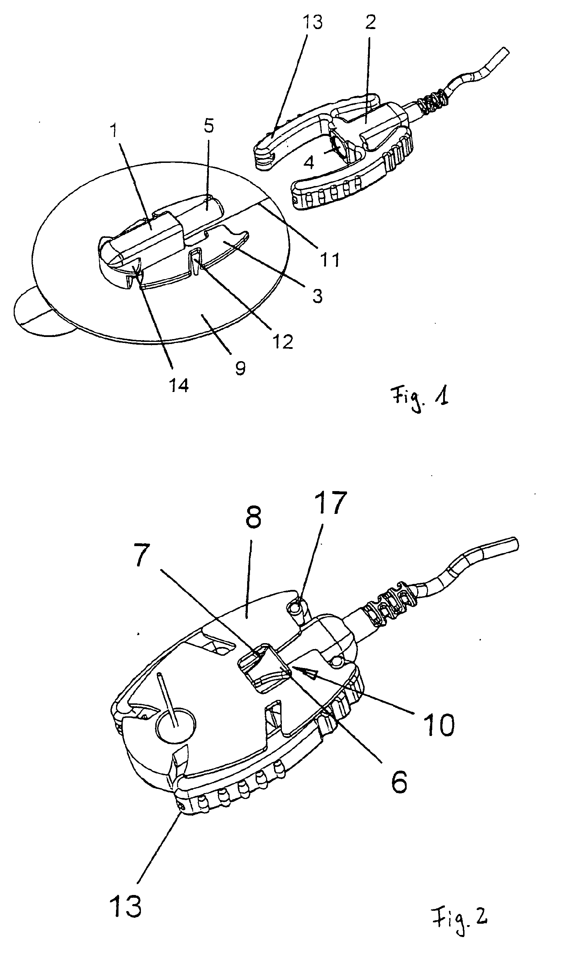

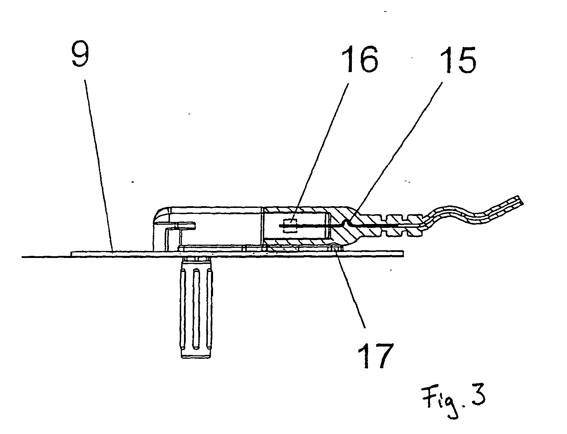

In den Figuren 1 bis 3 ist in verschiedenen Ansichten ein Ausführungsbeispiel für eine erfindungsgemäße Kupplungsvorrichtung dargestellt. Gleiche Elemente sind hierbei mit gleichen Bezugsziffern versehen.

Die erfindungsgemäße Kupplungsvorrichtung setzt sich im wesentlichen zusammen aus einem Katheteranschlussstück 1 eines Katheters und einem Kupplungsstück 2 an einer medizinischen Versorgungsleitung.

Das Katheteranschlussstück 1 ist auf einer Grundplatte 3 aufgesetzt, welche wiederum auf einer Klebeplatte 9 befestigt ist. Die Klebeplatte 9 wird im Einsatz des Katheters auf der Hautoberfläche des Patienten aufgeklebt und der Katheter so fixiert. Das Katheteranschlussstück 1 weist einen zylinderförmigen Zapfen 5 mit kreisförmigen Querschnitt auf. Dieser Zapfen 5 erstreckt sich in axialer Richtung parallel zur Ebene der Klebeplatte 9 und damit auch parallel zur Patientenoberfläche, wenn der Katheter zum Gebrauch auf der Patientenoberfläche befestigt ist. Im Innern des Katheters und des Katheteranschlussstückes 1 ist eine Leitung zum Einbringen einer zu verabreichenden Flüssigkeit, bspw. eines Medikamentes, geführt. Diese Leitung ist im vorliegenden Fall ein flexibler Schlauch, sie kann jedoch auch durch eine starre Stahlhohlnadel oder anderes gebildet sein. Die Leitung ist mit einem Ende in den Zapfen 5 hineingeführt und dort gegenüber der Umgebung mittels eines Dichtelementes 16 abgedichtet. Dieses Dichtelement 16 ist aus nachfolgend näher zu beschreibenden Gründen vorperforiert. Die Grundplatte 3 des Katheteranschlussstückes 1 weist in einem Bereich unterhalb des Zapfens 5 eine mit Hinterschnitten versehene Ausnehmung auf, die als Keilsitz dient. Dieser Keilsitz ist seitlich durch in der Grundplatte selbst gebildete Formschlusselemente 8 begrenzt. Die Formschlusselemente 8 sind mit dem Rest der Grundplatte 3 nur durch einen am Ende von von der Außenseite her in die Grundplatte 3 geführten Ausnehmungen 12 belassenen Materialstegen verbunden. Diese Materialstege bilden Gelenke, um die die Formschlusselemente 8 voneinander weg bzw. aufeinander zu bewegbar sind.

An dem dem Zapfen 5 gegenüber liegenden Ende des Katheteranschlussstückes 1 befinden sich seitlich Führungsstege 14. Diese sind linsenendförmig ausgebildet.

Das Kupplungsstück 2 weist zentral eine Buchse 4 mit kreisförmigem Querschnitt auf. Diese Buchse 4 ist zur Aufnahme des Zapfens 5 des Katheteranschlussstückes 1 ausgebildet und entsprechend dimensioniert. Über dünne Materialstege sind einstückig mit dem Kupplungsstück außenliegende Hebelarme 13 verbunden. Diese sind konvex gebogen und weisen an ihren dem Übergang des Kupplungsstückes 2 zu der medizinischen Versorgungsleitung gegenüberliegenden Ende jeweils Schlitze bzw. Nuten auf. An dem jeweils anderen, also der medizinischen Versorgungsleitung zugewandten Ende, weisen die Hebelarme 13 auf der beim Anschließen des Kupplungsstückes 2 an das Katheteranschlussstück 1 der Patientenoberfläche zugewandten, unteren Ende senkrecht nach unten weisende Steuerzapfen 17 auf. Auf derselben Unterseite weist das Katheteranschlussstück 2 zentral unterhalb der Buchse einen Verriegelungskeil 7 auf, der mit seinem keilförmig verbreiterten Ende dem Ende des Kupplungsstückes 2 zugewandt ist, an dem die medizinische Versorgungsleitung angelegt ist. Die Hebelarme 13 sind auf ihrer Außenseite mit einer Riffelung für ein besseres Angreifen versehen.

Im Innern des Kupplungsstückes 2 weist dieses eine mit der medizinischen Versorgungsleitung verbundene Kupplungskanüle 15 in Form einer metallenen Hohlnadel auf. Diese Kupplungskanüle 15 ist mit einem abgerundeten Punktionsende versehen. Die Kupplungskanüle 15 ist dabei in ihrer Erstreckung so lang, dass sie bei vollständig in die Buchse 4 eingeschobenem Zapfen 5 das Dichtelement 16 im Innern des Zapfens 5 durchdringen und somit eine Verbindung zwischen dem flexiblen Schlauch im Innern des Katheters und der medizinischen Versorgungsleitung herstellen kann.

Die erfindungsgemäße Kupplungsvorrichtung funktioniert folgendermaßen:
Zum Verbinden des Kupplungsstücks 2 mit dem Katheteranschlussstück 1 werden diese beiden Elemente zunächst wie in Fig. 1 dargestellt zueinander ausgerichtet. Dann wird das Kupplungsstück 2 mit der Buchse 4 über den Zapfen 5 des Katheteranschlussstücks 1 aufgeschoben. Hierbei erfährt das Kupplungsstück 2 bereits eine axiale Führung. Eine weitere Führung ergibt sich dadurch, dass die seitlich an dem Kupplungsstück 2 angeordneten Hebelarme 13 mit ihrer Unterseite auf der Grundplatte 3 gleiten, wodurch ein Verkanten des Kupplungsstückes 2 nicht möglich ist. Das Kupplungsstück 2 wird nun auf dem Katheteranschlussstück 1 axial geführt durch die Kombination von Zapfen 5 und Buchse 4 weiter voran geschoben, bis der Verriegelungskeil 6 mit seinen seitlichen Steuerkurven 7 die Formschlusselemente 8 der Grundplatte 3 erreicht. Mit den Steuerkurven 7 spreizt der Verriegelungskeil 6 die Formschlusselemente 8, welche dabei um die am Ende der Ausnehmungen 12 belassenen Materialstege auseinander biegen. Zum weiteren Aufschieben des Kupplungsstückes 2 auf das Katheteranschlussstück 1 gelangt der Verriegelungskeil 7 vollständig in den zwischen den Formschlusselementen 8 gebildeten Keilsitz, wobei die Formschlusselemente 8 "zurückschnappen" und den Verriegelungskeil 7 über die Höhe 10 der Hinterschnitte verriegeln. Der Verriegelungskeil 7 ist dabei in seiner Längserstreckung genau so lang, wie die Ausnehmung des Keilsitzes von der den Hinterschnitten gegenüberliegenden Stirnseite bis zu den Hinterschnitten. In dieser Position sind Hebelarme 13 mit ihren an den Enden ausgebildeten Nuten über die Führungsstege 14 geglitten, und die Steuerzapfen 17 greifen an am Ende der Formschlusselemente ausgebildeten Vorsprüngen an.

Beim Zurückschnappen der Formschlusselemente 8 entsteht ein hörbares Rastgeräusch, so dass der Anwender der Kupplungsvorrichtung weiß, dass das Kupplungsstück 2 und das Katheteranschlussstück 1 korrekt verriegelt sind. Zudem ist das Zurückschnappen der Formschlusselemente 8 taktil spürbar, so dass der Anwender auch auf diesem Wege ein Signal erhält, dass die Ankopplung des Kupplungsstückes 2 an das Katheteranschlussstück 1 korrekt abgeschlossen ist.

Beim Aufschieben des Kupplungsstückes 2 auf das Katheteranschlussstück 1 durchdringt auch die abgerundete Penetrationsspitze der Kupplungskanüle 15 das Dichtelement 16, welches zu diesem Zwecke vorperforiert ist.

Soll nun das Kupplungsstück 2 wieder von dem Katheteranschlussstück 1 getrennt werden, bspw. um einen neuen Katheter zu setzen und mit der medizinischen Versorgungsleitung zu verbinden, so werden hierzu die Hebelarme 13 an ihren an den Führungsstegen 14 liegenden Enden gegeneinander gedrückt. Dies bedingt ein Auseinanderspreizen der gegenüberliegenden Enden der Hebelarme 13 und der daran angeordneten Steuerzapfen 17. Dabei drücken die Steuerzapfen 17 die Formschlusselemente 8 auseinander, so dass der Verriegelungskeil 6 aus seinem Keilsitz freigegeben wird. Zugleich werden die vorderen Enden der Hebelarme 13 durch die Linsenendform der Führungsstege 14 in axialer Richtung (bezogen auf den Zapfen 5 bzw. die Buchse 4) zurückgeschoben, so dass bereits die Trennung von Zapfen 5 und Buchse 4 eingeleitet wird. Eine weitere Unterstützung dieser Trennungsbewegung wird durch das Zusammenspiel der Formschlusselemente 8 mit den Steuerkurven 7 des Verriegelungskeils 6 erreicht. Die Formschlusselemente 8 schnappen aufgrund der Federwirkung der am Ende der Ausnehmungen 12 belastenden Materialstege nämlich wieder aufeinander zu, sobald sich die Steuerzapfen 17 von den Vorsprüngen der Formschlusselemente 8 gelöst haben. Dabei wird der Verriegelungskeil 6, der inzwischen aus dem Keilsitz herausbewegt worden ist, aufgrund des Zusammenspiels der Formschlusselemente 8 und der Steuerflächen 7 weiter axial in Trennungsrichtung bewegt. Schließlich kann der Anwender die letzte Trennung von Kupplungsstück 2 und Katheteranschlussstück 1 durch Abziehen des Kupplungsstückes 2 vollziehen, das Kupplungsstück 2 steht dann zu einem erneuten Anschluss an dasselbe oder ein anderes Katheteranschlussstück 1 zur Verfügung.

Die beschriebene Kupplungsvorrichtung besticht durch eine einfache und sichere Handhabung, durch ein hörbares bzw. fühlbares Einrasten des Verriegelungskeils 6 in den Keilsitz und ein damit gegebenes Signal an den Anwender, dass die Verbindung korrekt hergestellt ist, sowie durch eine sichere Fixierung der Kupplungsverbindung in axialer Richtung (bezogen auf den Zapfen 5 und die Buchse 4) wie auch in Richtungen quer dazu.

### Bezugszeichenliste

- 1: Katheteranschlussstück
- 2: Kupplungsstück
- 3: Grundplatte
- 4: Buchse
- 5: Zapfen
- 6: Verriegelungskeil
- 7: Steuerkurve
- 8: Formschlusselement
- 9: Klebeplatte
- 10: Höhe
- 11: Einschnitt
- 12: Ausnehmung
- 13: Hebelarm
- 14: Führungssteg
- 15: Kupplungskanüle
- 16: Dichtelement
- 17: Steuerzapfen

## Patentansprüche

1. Kupplungsvorrichtung zum Anschließen einer medizinischen Versorgungsleitung an einen Katheter mit einem an dem Katheter angeordneten Katheteranschlussstück (Hub) (1) und einem an einem Anschlussende der medizinischen Versorgungsleitung zum Zusammenwirken mit dem Katheteranschlussstück angeordneten Kupplungsstück (2), wobei jeweils eines der Elemente Katheteranschlussstück und Kupplungsstück (2) jeweils eines der paarweise zusammenwirkenden Kupplungselemente aufweist:
1. einen axial in einer Aufschubrichtung des Kupplungsstückes ausgerichteten Zapfen (5) bzw. eine zur Aufnahme des Zapfens (5) ausgestaltete Buchse (4) und,
2. einen in einer parallel zur axialen Erstreckungsrichtung des Zapfens (5) keilförmig ausgebildeten Verriegelungskeil (6) bzw. einen Keilsitz zur formschlüssigen Aufnahme des Verriegelungskeils (6),
**dadurch gekennzeichnet, daß** der Keilsitz durch gelenkig gelagerte Formschlusselemente (8) begrenzt ist, welche zum Lösen einer formschlüssigen Verbindung zwischen dem Verriegelungskeil (6) und dem Keilsitz auseinander bewegbar sind und wobei an dem Element, Katheteranschlussstück (1), bzw. Kupplungsstück (2), an dem der Verriegelungskeil (6) angeordnet ist voneinander beabstandete Hebelarme (13) angeordnet sind, die bei zusammengefügter Kupplungsvorrichtung so mit den Formschlusselementen (8) zusammenwirken, dass sie diese bei Betätigung der Hebelarme spreizen und den Verriegelungskeil (6) freigeben.

2. Kupplungsvorrichtung nach Anspruch 1, **dadurch gekennzeichnet, dass** die Hebelarme (13) an dem Element, an dem sie angeordnet sind, einstückig ausgebildet und durch ein durch einen jeweils zwischen dem Enden des Hebelarms (13) ausgebildeten, den Hebelarm (13) mit dem restlichen Element verbindenden und in zwei Abschnitte unterteilenden, schmalen Materialsteg gebildetes Filmgelenk als zweiarmige Hebelarme (13) ausgebildet sind, wobei zumindest bei zusammengefügter Kupplungsvorrichtung jeweils ein erster Abschnitt des Hebelarms (13) zum Zusammenwirken mit einem der Formschlusselemente (8) an diesem angreift und ein zweiter Abschnitt des Hebelarms (13) der Betätigung der Hebelarme (13) dient.

3. Kupplungsvorrichtung nach Anspruch 2, **dadurch gekennzeichnet, dass** die Hebelarme (13) an ihren ersten Abschnitten jeweils einen in einer Kupplungsstellung nach unten, in Richtung der Patientenoberfläche, weisenden Steuerzapfen (17) aufweisen, die bei zusammengefügter Kupplungsvorrichtung an entsprechenden Gegenstücken in den Formschlusselementen (8) zum Spreizen derselben bei Betätigung der zweiten Abschnitte der Hebelarme (13) angreifen.

4. Kupplungsvorrichtung nach einem der Ansprüche 1 bis 3, **dadurch gekennzeichnet, dass** die Hebelarme (13) an den äußeren Enden ihrer zweiten Abschnitte Nuten aufweisen, die bei zusammengefügter Kupplungsvorrichtung an Führungsstegen (14) angreifen, die an dem Elemente, Katheteranschlussstück (1), bzw. Kupplungsstück (2) ohne die Hebelarme (13) angeordnet sind und die eine solche Kontur aufweisen, dass bei Betätigung der Hebelarme (13) die Nuten auf den Führungsstegen (14) so geführt werden, dass neben dem Spreizen der Formschlusselemente (8) eine in axialer Richtung des Zapfens (5), bzw. der Buchse (4) in eine Trennungsrichtung weisende Kraft auf das Element ausgeübt wird, an dem die Hebelarme (13) angeordnet sind.

5. Kupplungsvorrichtung nach einem der Ansprüche 1 bis 4, **dadurch gekennzeichnet, dass** die Buchse (4) und der Verriegelungskeil (6) an dem Kupplungsstück (2) und der Zapfen (5) und die Formschlusselemente (8) an dem Katheteranschlussstück (1) angeordnet sind, wobei die axiale Erstreckung des Zapfens (5) auf dem Katheteranschlussstück im wesentlichen parallel zu einer auf der Hautoberfläche des Patienten aufzusetzenden Unterseite des Katheteranschlussstückes (1) verläuft.

6. Kupplungsvorrichtung nach Anspruch 5, **dadurch gekennzeichnet, dass** die an dem Kupplungsstück (2) angeordneten Hebelarme (13) in einer Ausrichtung zum Zusammenfügen der Elemente, Katheteranschlussstück (1) und Kupplungsstück (2), seitlich an dem Kupplungsstück (2) abstehen und bei zusammengefügter Kupplungsvorrichtung das Katheteranschlussstück sichelförmig umgreifend auf einer Grundplatte (3) des Katheteranschlussstückes aufliegen.

7. Kupplungsvorrichtung nach Anspruch 6, **dadurch gekennzeichnet, dass** der Verriegelungskeil (6) auf einer in der Ausrichtung zum Zusammenführen der Elemente der Patientenoberfläche zugewandten Unterseite des Kupplungsstückes angeordnet ist und dass die Formschlusselemente (8) als Teile der Grundplatte (3) ausgebildet sind, die über durch Materialverdünnungen bzw. Ausnehmungen (12) in der Grundplatte (3) gegeneinander und gegenüber dem Rest der Grundplatte (3) gelenkig gelagert sind.

8. Kupplungsvorrichtung nach Anspruch 7, **dadurch gekennzeichnet, dass** das Katheteranschlussstück (1) mit seiner Grundplatte (3) auf einer Klebeplatte (9) befestigt ist, welche im Bereich der Formschlusselemente (8) einen Einschnitt (11) aufweist, um eine Aufspreizbewegung der Formschlusselemente (8) zu ermöglichen.

9. Kupplungsvorrichtung nach einem der Ansprüche 1 bis 8, **dadurch gekennzeichnet, dass** in dem bzw. der an dem Katheteranschlussstück angeordneten Zapfen (5), bzw. Buchse (4) ein vorperforiertes Dichtelement (Septum) (16) angeordnet ist, das ein in dem Katheteranschlussstück (1) liegendes Leitungselement nach außen abdichtet, und dass in der bzw. dem an dem Kupplungsstück (2) angeordneten Buchse (4), bzw. Zapfen (5) eine Kupplungskanüle (15) mit abgerundeter Penetrationsspitze angeordnet ist, die bei zusammengefügter Kupplungsvorrichtung das Dichtelement durchstößt.

10. Kupplungsvorrichtung nach einem der Ansprüche 1 bis 9, **dadurch gekennzeichnet, dass** der Zapfen (5) zylinderförmig ausgebildet und die Buchse (4) mit einer entsprechenden Aufnahmeöffnung versehen ist.

## Claims

1. Coupling device for the connection of a medical supply line to a catheter with a catheter connecting piece (lift) (1) placed on the catheter and a coupling piece (2) placed on a connection end of the medical supply line for cooperating with the catheter connecting piece, in each case one of the elements catheter connecting piece and coupling piece (2) having one of the pairwise cooperating coupling elements:
1) a pin (5) oriented axially in a push-up direction of the coupling piece or a bush (4) designed for receiving the pin (5) and
2) a wedge-shaped locking key (6) parallel to the axial extension direction of pin (5) or a key seat for the positive reception of the locking key (6), **characterized in that** the key seat is bounded by articulatedly mounted interlocking elements (8), which can be moved apart for releasing a positive connection between locking key (6) and the key seat and in which on the catheter connecting piece (1) or coupling piece (2) on which the locking key (6) is placed are provided mutually spaced lever arms (13), which when the coupling device is joined together cooperate with the interlocking elements (8) in such a way that they are forced apart by the operation of the lever arms and free the locking key (6).

2. Coupling device according to claim 1, **characterized in that** the lever arms (13) are integrally constructed on the element on which they are placed and are constructed as two-armed lever arms (13) by a narrow material web connecting lever arm (13) to the remaining element and subdividing same into two portions and constructed between the ends of lever arm (13) and at least when the coupling device is fixed together in each case a first portion of the lever arm (13) acts thereon for cooperating with one of the interlocking elements (8) and a second portion of lever arm (13) is used for operating the lever arms (13).

3. Coupling device according to claim 2, **characterized in that** on their first portions the lever arms (13) have in each case a control pin (17) directed downwards in a coupling position towards the patient surface and when the coupling device is fixed together engaging on corresponding counterparts in the interlocking elements (8) for forcing the same apart on operating the second portions of lever arms (13).

4. Coupling device according to one of the claims 1 to 3, **characterized in that** on the outer ends of their second portions the lever arms (13) have grooves acting on guide webs (14) when the coupling device is fixed together, said webs (14) being located on the catheter connecting piece (1) or coupling piece (2) without the lever arms (13) and which have a contour such that on operating the lever arms (13) the grooves on guide webs (14) are guided in such a way that, apart from forcing apart the interlocking elements (8), they exert in a separating direction a force on the element on which the lever arms (13) are placed in the axial direction of pin (5) or bush (4).

5. Coupling device according to one of the claims 1 to 4, **characterized in that** the bush (4) and locking key (6) are located on coupling piece (2) and pin (5) and the interlocking elements (8) on catheter connecting piece (1), the axial extension of pin (5) on the catheter connecting piece being substantially parallel to an underside of the catheter connecting piece (1) to be placed on the skin surface of the patient.

6. Coupling device according to claim 5, **characterized in that** the lever arms (13) located on the coupling piece (2) project laterally on coupling piece (2) in an orientation for joining together the catheter connecting piece (1) and coupling piece (2) and when the coupling device is fixed together rest on a base plate (3) of the catheter connecting piece embracing in crescent-shaped manner said catheter connecting piece.

7. Coupling device according to claim 6, **characterized in that** the locking key (6) is located on an underside of the coupling piece facing the patient surface in an orientation for bringing together the elements and that the interlocking elements (8) are constructed as parts of base plate (3), which are articulatedly mounted against one another and the remainder of the base plate (3) by material thinnings or recesses (12) in said base plate (3).

8. Coupling device according to claim 7, **characterized in that** the base plate (3) of catheter connecting piece (1) is fixed to an adhesive plate (9), which in the vicinity of the interlocking elements (8) has a notch (11) for permitting an expanding movement of interlocking elements (8).

9. Coupling device according to one of the claims 1 to 8, **characterized in that** a preperforated sealing element (septum) (16) is placed in the pin (5) or bush (4) located on the catheter connecting piece and seals to the outside a line element located in the catheter connecting piece (1), and that in the bush (4) or pin (5) located on coupling piece (2) is provided a coupling cannula (15) with a rounded penetration tip which perforates the sealing element when the coupling device is fixed together.

10. Coupling device according to one of the claims 1 to 9, **characterized in that** the pin (5) is cylindrical and the bush (4) is provided with a corresponding reception opening.

## Revendications

1. Dispositif d'accouplement pour raccorder un conduit médical d'alimentation à un cathéter, comprenant une pièce de raccordement (1) (course) située sur le cathéter, et une pièce d'accouplement (2) située à l'extrémité de raccordement du conduit médical d'alimentation, en vue de coopérer avec ladite pièce de raccordement du cathéter, sachant que l'un considéré des éléments, matérialisés par la pièce de raccordement du cathéter et par la pièce d'accouplement (2), est respectivement muni des éléments d'accouplement coopérant par paires :
1. d'un tenon (5) orienté axialement dans une direction d'enfilement de la pièce d'accouplement ou, respectivement, d'une douille (4) conçue pour recevoir ledit tenon (5), et
2. d'une cale de verrouillage (6), de réalisation cunéiforme parallèlement à la direction d'étendue axiale du tenon (5) ou, respectivement, d'un siège cunéiforme pour recevoir ladite cale de verrouillage (6) par concordance de formes,
**caractérisé par le fait que** le siège cunéiforme est délimité par des éléments (8) à complémentarité de formes qui sont montés de manière articulée et peuvent être éloignés mutuellement, afin de supprimer une liaison par concordance de formes entre la cale de verrouillage (6) et le siège cunéiforme, sachant que l'élément, respectivement matérialisé par la pièce (1) de raccordement du cathéter ou par la pièce d'accouplement (2), sur lequel la cale de verrouillage (6) est disposée, comporte des bras de levier (13) mutuellement espacés qui coopèrent avec les éléments (8) à complémentarité de formes lorsque le dispositif d'accouplement est assemblé, de façon telle qu'ils écartent lesdits éléments lors d'un actionnement des bras de levier, et libèrent la cale de verrouillage (6).

2. Dispositif d'accouplement selon la revendication 1, **caractérisé par le fait que** les bras de levier (13) sont réalisés d'un seul tenant avec l'élément sur lequel ils se trouvent, et sont conçus comme des bras de levier (13) à deux bras, avec interposition d'une articulation pelliculaire formée d'une étroite membrure de matière qui est respectivement façonnée entre les extrémités du bras de levier (13), relie ledit bras de levier (13) au reste de l'élément, et est scindée en deux tronçons, sachant que, au moins lorsque le dispositif d'accouplement est assemblé, un premier tronçon respectif du bras de levier (13) vient en prise avec l'un des éléments (8) à complémentarité de formes, en vue de coopérer avec celui-ci, et un second tronçon dudit bras de levier (13) sert à l'actionnement des bras de levier (13).

3. Dispositif d'accouplement selon la revendication 2, **caractérisé par le fait que** les bras de levier (13) sont respectivement pourvus, sur leurs premiers tronçons, d'une cheville de commande (17) qui, dans une position d'accouplement, est tournée vers le bas en direction de la surface cutanée du patient et qui, lorsque le dispositif d'accouplement est assemblé, vient en prise avec des pièces complémentaires correspondantes, dans les éléments (8) à complémentarité de formes, en vue d'écarter ces derniers lors d'un actionnement des seconds tronçons des bras de levier (13).

4. Dispositif d'accouplement selon l'une des revendications 1 à 3, **caractérisé par le fait que** les bras de levier (13) comportent, aux extrémités extérieures de leurs seconds tronçons, des rainures venant en prise, lorsque le dispositif d'accouplement est assemblé, avec des membrures de guidage (14) qui se trouvent sur l'élément respectivement matérialisé par la pièce (1) de raccordement du cathéter ou par la pièce d'accouplement (2), sans les bras de levier (13), et qui possèdent un profil tel que, lors d'un actionnement des bras de levier (13), lesdites rainures soient guidées, sur lesdites membrures de guidage (14), de telle sorte que, outre l'écartement des éléments (8) à complémentarité de formes, une force orientée dans la direction axiale considérée du tenon (5) ou de la douille (4), dans le sens d'une séparation, soit appliquée à l'élément sur lequel les bras de levier (13) sont disposés.

5. Dispositif d'accouplement selon l'une des revendications 1 à 4, **caractérisé par le fait que** la douille (4) et la cale de verrouillage (6) sont placées sur la pièce d'accouplement (2), et le tenon (5) et les éléments (8) à complémentarité de formes sont placés sur la pièce (1) de raccordement du cathéter, l'étendue axiale dudit tenon (5), sur ladite pièce de raccordement du cathéter, s'étendant pour l'essentiel parallèlement à une face inférieure de ladite pièce (1) de raccordement du cathéter, devant être plaquée contre la surface cutanée du patient.

6. Dispositif d'accouplement selon la revendication 5, **caractérisé par le fait que** les bras de levier (13) situés sur la pièce d'accouplement (2) font saillie latéralement sur ladite pièce d'accouplement (2), selon une orientation visant à l'assemblage des éléments matérialisés par la pièce (1) de raccordement du cathéter et la pièce d'accouplement (2), et sont appliqués sur une platine d'embase (3) de ladite pièce de raccordement du cathéter, lorsque le dispositif d'accouplement est assemblé, en ceinturant ladite pièce de raccordement du cathéter à la manière d'un croissant.

7. Dispositif d'accouplement selon la revendication 6, **caractérisé par le fait que** la cale de verrouillage (6) se trouve sur une face inférieure de la pièce d'accouplement qui, dans l'orientation visant à l'assemblage des éléments, est tournée vers la surface cutanée du patient ; et **par le fait que** les éléments (8) à complémentarité de formes sont réalisés comme des parties de la platine d'embase (3) qui sont montées de manière articulée, l'une vis-à-vis de l'autre et vis-à-vis du reste de ladite platine d'embase (3), grâce à des rétrécissements de matière ou à des évidements (12) respectivement façonnés dans ladite platine d'embase (3).

8. Dispositif d'accouplement selon la revendication 7, **caractérisé par le fait que** la pièce (1) de raccordement du cathéter est fixée, par sa platine d'embase (3), sur une plaquette adhésive (9) présentant une entaille (11), dans la région des éléments (8) à complémentarité de formes, afin d'autoriser un mouvement d'écartement desdits éléments (8) à complémentarité de formes.

9. Dispositif d'accouplement selon l'une des revendications 1 à 8, **caractérisé par** la présence respective, dans le tenon (5) ou dans la douille (4) situé(e) sur la pièce de raccordement du cathéter, d'un élément préperforé d'étanchement (septum) (16) assurant l'étanchéité, vers l'extérieur, d'un élément de conduit logé dans ladite pièce (1) de raccordement du cathéter ; et par le fait que la douille (4) ou le tenon (5), respectivement situé(e) sur la pièce d'accouplement (2), renferme une canule d'accouplement (15) à pointe de pénétration arrondie, qui perfore l'élément d'étanchement lorsque le dispositif d'accouplement est assemblé.

10. Dispositif d'accouplement selon l'une des revendications 1 à 9, **caractérisé par le fait que** le tenon (5) est de réalisation cylindrique, et la douille (4) est dotée d'un orifice récepteur correspondant.
